# DEMANDE DE BREVET EUROPEEN

(11) **EP 0 773 004 A1**
(43) Date de publication de la demande: **14.05.1997**
(21) Numéro de dépôt: 95420309.7
(22) Date de dépôt: 07.11.1995
(51) Int. Cl.: A61B 17/80

(54) **Plaque d'ostéotomie d'ouverture**

(71) Demandeur: IMPLANTS ORTHOPEDIQUES TOUTES APPLICATIONS, S.A.R.L. dite:, 42000 Saint-Etienne (FR)
(72) Inventeur: Deaux, Pierre G., F-42000 Saint Etienne (FR)
(74) Mandataire: Perrier, Jean-Pierre

(57) **Abrégé**

Cette plaque est composée de deux éléments munis chacun d'une patte de fixation sur l'élément correspondant de l'os et de crans aptes à coopérer avec ceux de l'autre élément pour assurer un calage en translation de ces éléments.

Selon l'invention, les deux éléments sont en forme de mâchoires entrecroisées (A, B) comportant chacune, d'une part, au moins un bec coudé (8, 17) s'insérant entre les faces réséquées, le bec de chaque mâchoire s'appuyant sur une face réséquées différente de celle contre laquelle s'appuie le bec de l'autre mâchoire, et, d'autre part, des crans (12, 16) qui, saillants des chants faisant vis à vis à ceux de l'autre mâchoire, coopérèrent avec les crans de cette mâchoire pour régler l'écartement des becs et des faces réséquées.

## Description

L'invention est relative à une plaque d'ostéotomie d'ouverture.

En présence d'une malformation ou d'une déformation d'un membre inférieur, affectant la stabilité ou la mobilité, il est connu de redresser le membre, par exemple le tibia, en procédant à une opération chirurgicale consistant :
- à réaliser sous la tête de l'os, une coupe transversaie puis à écarter les fragments pour former, entre les deux faces, un angle dépendant du besoin de redressement et compris en général entre quelques degrés et 20 °,
- et à caler les faces délimitées par les coupes en interposant entre elles des cales osseuses.

Cette technique d'ostéoplastie est imprécise lorsqu'elle est réalisée et procure des résultats instables dans le temps puisque, sous le poids du corps comprimant les membres inférieurs, les faces en vis à vis ont tendance en se rapprochant à comprimer les cales osseuses et à réduire l'angulation réparatrice.

La mise en place d'une plaque reliant les deux éléments de l'os apporte une stabilité dans l'angulation mais ne résout pas le problème de la précision de cette angulation au moment de l'ostéoplastie.

Il en est de même avec la plaque décrite dans DE-C-867 422 qui est composée de deux éléments se fixant chacun sur l'un des éléments de l'os et comportant des crans complémentaires de blocage saillant des faces en vis à vis de leurs extrémités se superposant.

L'invention a pour objet de fournir une plaque d'ostéotomie d'ouverture en deux éléments garantissant la précision de l'angulation, tant lors de l'opération que dans le temps.

A cet effet, dans la plaque selon l'invention, les deux éléments sont en forme de mâchoires entrecroisées comportant chacune, d'une part, au moins un bec coudé s'insérant entre les faces réséquées, le bec de chaque mâchoire s'appuyant sur une face réséquées différente de celle contre laquelle s'appuie le bec de l'autre mâchoire, et, d'autre part, des crans qui, saillants des chants faisant vis à vis à ceux de l'autre mâchoire, coopérèrent avec les crans de cette mâchoire pour régler l'écartement des becs et des faces réséquées.

Avec ce dispositif, lorsque le chirurgien a réalisé la coupe et écarté les fragments osseux, il fixe l'une des mâchoires sur la tête de l'os, l'autre mâchoire sur le fût du même os, en prenant soin de plaquer chacun des becs correspondant contre la face réséquée, puis il règle l'angle d'ouverture formé entre les deux faces réséquées en déplaçant l'une des mâchoires par rapport à l'autre, soit dans le sens de l'écartement, soit dans le sens de leur rapprochement. Lorsque l'angle recherché est atteint, le calage définitif est assuré en engageant les crans de l'une des mâchoires dans les crans de l'autre mâchoire.

Il est évident que la précision du réglage dépend du pas des crans qui sont eux-mêmes choisis pour donner des variations d'angulation de l'ordre du degré.

Lorsque le patient s'appuie sur sa jambe ainsi traitée, les deux éléments de la plaque d'ouverture transmettent les efforts perçus par la tête au fût de l'os en maintenant l'écartement de ces deux éléments et en s'opposant à toute modification de leur position relative.

Dans une forme d'exécution de l'invention, une première mâchoire présente, en vue de dessus, la forme générale d'un "U" dont les ailes sont munies de crans, au moins sur une partie de leur face interne, et comportent, à chacune de leurs extrémités, un coude formant bec d'écartement, tandis que la seconde mâchoire est composée d'une tige qui, apte à s'engager entre les ailes de la première mâchoire, est munie, d'une part et sur au moins une partie de sa longueur, de crans saillant de ses faces latérales et, d'autre part et près de son extrémité munie de la patte de fixation, d'un tenon en saillie formant bec d'écartement.

Lorsque la seconde mâchoire est montée entre les ailes de la première mâchoire, son bec d'écartement est disposé dans le plan médian de la plaque, tandis que les becs d'écartement de la première mâchoire sont disposés de part et d'autre de ce plan.

D'autres caractéristiques et avantages ressortiront de la description qui suit en référence au dessin schématique annexé représentant, à titre d'exemple non limitatif, une forme d'exécution de la plaque d'ostéotomie d'ouverture selon l'invention.
Figure 1 est une vue en perspective représentant les deux éléments de la plaque,
Figure 2 est une vue en plan par dessus montrant les deux éléments assemblés,
Figures 3 et 4 sont des vues partielles de côté montrant la plaque lorsqu'elle est posée et pour deux angulations correctives différentes,
Figures 5 et 6 sont des vues en coupe transversale suivant V-V de figure 2 de chacune des deux mâchoires.

Cette plaque est composée de deux mâchoires, respectivement A et B. La première mâchoire A présente, en vue par dessus, la forme générale d'un "U", c'est à dire comporte deux ailes 2 reliées par une âme 3 et délimitant entre elles un logement 4. Chacune des ailes comporte, à proximité de l'âme 3, une excroissance externe 5, constituant patte de fixation et traversée par un alésage 6 avec un lamage boule 7. A son extrémité libre, chacune des ailes est munie d'un retour coudé 8 formant bec d'écartement. Chaque bec est muni d'un chanfrein 9 favorisant l'introduction de l'autre mâchoire dans le logement 4. Enfin, près de l'âme 3 et en saillie de son chant interne 10, chaque aile 2 comporte des crans transversaux 12 répartis avec un pas constant ayant une valeur de l'ordre de 1 mm.

Dans la forme d'exécution représentée et pour s'adapter à la forme de l'os, les extrémités libres des ailes 2 sont légèrement pliées vers le haut en 2a.

La seconde mâchoire B est composée d'une tige 13 dont une extrémité est solidaire d'une patte de fixation 14 comportant deux alésages transversaux 6 avec lamage boule 7. Cette tige a une largeur déterminée pour s'engager librement dans le logement 4 entre les ailes 2 de la mâchoire A. A son extrémité opposée à la patte de fixation et en saillie de chacun de ses chants latéraux 15, la tige 13 comporte une série de crans 16 ayant le même profil et le même pas que les crans 12 de la mâchoire A avec lesquels ils sont destinés à coopérer pour positionner longitudinalement les deux mâchoires. Cette tige comporte encore, dans sa partie qui est disposée à proximité de la patte de fixation 14, un bec d'écartement 17 saillant de sa face inférieure et disposé sensiblement dans son plan médian longitudinal.

Pour s'adapter à la forme de l'os, la mâchoire B est coudée en 13a et contrecoudée en 13b.

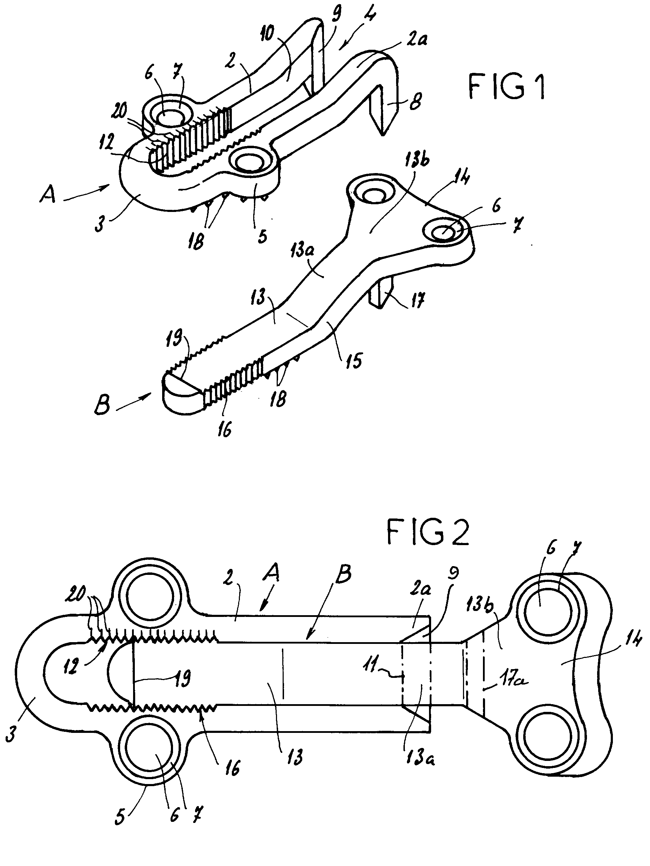

Les faces des deux mâchoires qui sont inférieures à la figure 1 et qui sont destinées à venir en contact avec l'os comportent, au moins localement, des picots 18 favorisant leur ancrage dans l'os.

Dans une forme d'exécution, l'une des deux mâchoires, et par exemple, celle B comporte, à proximité de son extrémité, un index 19 coopérant avec une graduation 20 ménagée sur la mâchoire A et indiquant la valeur d'écartement des faces actives des becs d'écartement 8 et 17, cette valeur d'écartement étant donnée en degré et éventuellement en millimètre.

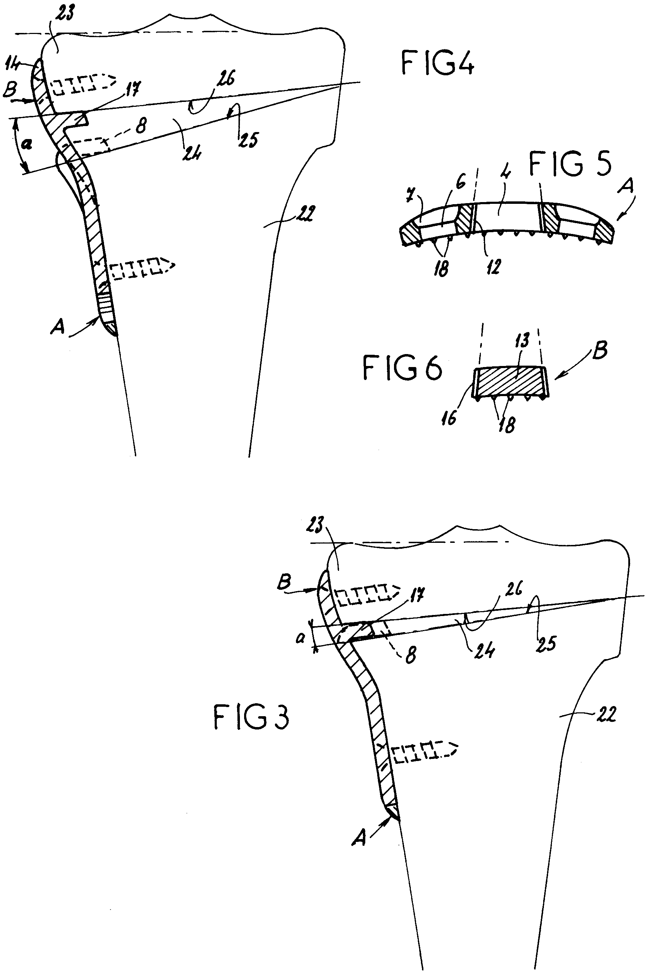

Suivant une autre caractéristique de l'invention, et comme montré plus en détails aux figures 5 et 6, la tige 13 de la mâchoire B présente, en section transversale, une forme trapézoïdale et plus précisément comporte des faces latérales qui vont en convergeant à l'opposé de la face venant en contact avec l'os. De même, le logement 4 de la première mâchoire A présente une section transversale trapézoïdale et comporte donc des faces internes 10 qui vont en convergeant à l'opposé de la face de cette mâchoire venant en contact avec l'os.

Avec cet agencement, lorsque la première mâchoire A est mise en place sur la mâchoire B, elle assure, par coopération des crans 12 et 16, le calage en translation longitudinale des deux mâchoires, et par la forme trapézoïdale de son logement 4, le calage en translation transversale vers l'extérieur de la mâchoire B.

Comme montré aux figures 3 et 4, les pattes de fixation 5 de la mâchoire A se fixent sur le fût 22 de l'os, tandis que la patte de fixation de la mâchoire B se fixe sur la tête de l'os 23. Dans ces conditions, les becs d'écartement respectivement 8 et 17 s'insèrent dans la fente 24 formée par la coupe osseuse et viennent chacun en appui contre les faces réséquées respectivement 25 et 26.

Cette plaque d'ostéotomie d'ouverture permet de régler précisément et de maintenir dans le temps l'angle d'ouverture a formé entre les faces réséquées 25 et 26 a une valeur constante allant d'une valeur de l'ordre de 3°30, dans le cas représenté à la figure 3 correspondant au plus faible angle d'ouverture tolérant le passage des becs 8 et 17, jusqu'à une valeur de l'ordre de 10° représentée à la figure 4, cette dernière valeur pouvant aller jusqu'à 20° avec un autre modèle de plaque.

Dans une variante de réalisation et pour réduire la pression d'appui des becs sur les fragments osseux, les becs 8 de la première mâchoire A sont reliés par un pontet transversal 11 représenté en traits mixtes figure 2, et disposé au-dessous des ailes 2 et le bec 17 de la seconde mâchoire B forme une barrette transversale 17a, montrée en traits mixtes à la même figure, dans la zone coudée 13b d'éloignement de la tête de fixation 14.

Il ressort de ce qui précède que la plaque selon l'invention améliore non seulement la précision du redressement mais procure aussi une fiabilité qui jusqu'à présent ne pouvait pas être atteinte.

## Revendications

1. Plaque d'ostéotomie d'ouverture composée de deux éléments munis chacun d'une patte de fixation sur l'élément correspondant de l'os et de crans aptes à coopérer avec ceux de l'autre élément pour assurer un calage en translation de ces éléments, **caractérisée en ce que** les deux éléments sont en forme de mâchoires entrecroisées (A-B) comportant chacune, d'une part, au moins un bec coudé (8, 17) s'insérant entre les faces réséquées (25, 26), le bec de chaque mâchoire s'appuyant sur une face réséquées (25, 26) différente de celle contre laquelle s'appuie le bec de l'autre mâchoire, et, d'autre part, des crans (12, 16) qui, saillants des chants faisant vis à vis à ceux de l'autre mâchoire, coopérèrent avec les crans de cette mâchoire pour régler l'écartement des becs et des faces réséquées.

2. Plaque selon la revendication 1, **caractérisée en ce qu'**une première mâchoire (A) présente, en vue de dessus, la forme générale d'un "U" dont les ailes (2) sont munies de crans (12), au moins sur une partie de leur face interne (10), et comportent, à chacune de leurs extrémités, un coude formant bec d'écartement (8), tandis que la seconde mâchoire (B) est composée d'une tige (13) qui, apte à s'engager entre les ailes (2) de la première mâchoire (A), est munie, d'une part et sur au moins une partie de sa longueur, de crans (16) saillant de ses faces latérales et, d'autre part et près de son extrémité munie de la patte de fixation (14), d'un tenon en saillie formant bec d'écartement (17).

3. Plaque selon l'une quelconque des revendications 1 et 2, **caractérisée en ce que** les crans (16) de l'une (B) des mâchoires sont associés à un index transversal (19) coopérant avec des graduations (20) portées sur l'autre mâchoire (A).

4. Plaque selon l'une quelconque des revendications 1 à 3, **caractérisée en ce que** la tige (13) de la seconde mâchoire B présente, en section transversale, une forme trapézoïdale dont les faces latérales convergent à l'opposé de sa face d'appui avec l'os, tandis que le logement (4) ménagé entre les ailes (2) de la première mâchoire (A) présente également, en section transversale, une forme trapézoïdale apte à caler en translation transversale la tige (13) de la première mâchoire (A).

5. Plaque selon l'une quelconque des revendications 1 à 4, **caractérisée en ce que** les ailes de la mâchoire en "U" (A) sont coudées et contrecoudées à proximité de leurs extrémités portant les becs (8), tandis que la mâchoire (B) est coudée et contrecoudée à proximité de son extrémité munie de la patte de fixation (14).

6. Plaque selon l'une quelconque des revendications 1 à 5, **caractérisée en ce que** des picots (18) d'ancrage dans l'os font saillies des faces des mâchoires (A et B) destinées à venir en contact avec l'os.
